# FASCICULE DE BREVET EUROPEEN

(11) **EP 0 918 854 B1**
(45) Date de publication et mention de la délivrance du brevet: **27.10.2004**
(21) Numéro de dépôt: 97937621.7
(22) Date de dépôt: 12.08.1997
(51) Int. Cl.: C12N 15/11, C12Q 1/68, C07K 14/35

(54) **FRAGMENTS D'ACIDES NUCLEIQUES SPECIFIQUES DE MYCOBACTERIES MEMBRES DU COMPLEXE M. TUBERCULOSIS) ET LEURS APPLICATIONS POUR LA DETECTION ET LE DIAGNOSTIC DIFFERENTIEL DES MEMBRES DU COMPLEXE M. TUBERCULOSIS**
NUKLEINSÄURE-FRAGMENTE, DIE SPEZIFISCHE FÜR DIE MITGLIEDER DES M. TUBERCULOSIS KOMPLEXES SIND, DEREN ANWENDUNG ZUR DETEKTION UND DIFFERENTIALE DIAGNOSE DER MITGLIEDER DES M. TUBERCULOSIS KOMPLEXES
M. TUBERCULOSIS COMPLEX MEMBERS MYCOBACTERIA SPECIFIC NUCLEIC ACID FRAGMENTS AND THEIR APPLICATIONS FOR THE DETECTION AND DIFFERENTIAL DIAGNOSIS OF M. TUBERCULOSIS COMPLEX MEMBERS

(30) Priorité: 19.08.1996 FR 9610277
(43) Date de publication de la demande: 02.06.1999
(73) Titulaire: Institut Pasteur de Lille, 59019 Lille Cedex (FR); INSTITUT NATIONAL DE LA SANTE ET DE LA RECHERCHE MEDICALE (INSERM), 75654 Paris Cédex 13 (FR)
(72) Inventeur: MAGDALENA, Juana, F-59000 Lille (FR); SUPPLY, Philip, B-7500 Tournai (BE); LOCHT, Camille, F-59830 Wannehain (FR)
(74) Mandataire: Bernasconi, Jean Raymond
(86) Numéro de dépôt international: PCT/FR1997/001483
(87) Numéro de publication internationale: WO 1998/007847

(56) Documents cités:
- WO-A-90/10085
- DATABASE EMBL: ENTRY MTCY130 Accession number Z73902, 29 mai 1996 XP002050042
- DATABASE EMBL: ENTRY MTCY02B10 Accession number Z75555, 30 juin 1996 XP002050043
- DATABASE EMBL: ENTRY MTCY20G9 Accession number Z77162, 18 Septembre 1996 XP002050038
- SUPPLY P. AND LOCHT C.: "Cloning of a complete two-component regulatory system of Mycobacterium bovis BCG" JOURNAL OF CELLULAR BIOCHEMISTRY, vol. 19B, 5 février 1995 - 15 mars 1995, page 79 XP002050037
- WREN ET AL.: "Degenerate PCR primers for the amplification of fragments from genes encoding response regulators from a range of pathogenic bacteria" FEMS MICROBIOLOGY LETTERS, vol. 99, no. 2+3, 1 décembre 1992, pages 287-291, XP000647546 cité dans la demande

## Description

La présente invention concerne des séquences d'acides nucléiques de mycobactéries appartenant au complexe *M*. *tuberculosis*.

L'invention concerne également une méthode de détection in vitro de souches de mycobactéries appartenant au complexe *M. tuberculosis* ainsi qu'une méthode pour un diagnostic différentiel de souches du complexe *M. tuberculosis,* notamment pour différencier la présence du BCG de celle des autres membres du complexe dans un échantillon.

Approximativement, 1,7 milliard de personnes soit 1/3 de la population mondiale sont infectées par *M. tuberculosis* (Sudre et al., 1992). En 1990, le nombre de cas de tuberculose estimé était de 8 millions, dont 2,9 millions de morts (Sudre et al., 1992). Ces dernières années, le nombre de cas de tuberculose aux Etats-unis et en Europe a augmenté de 3 à 6 % par an, principalement dans des populations à haut risque telles que les patients souffrant de sida, les alcooliques chroniques, les sans-abris et les toxicomanes (Barnes et al., 1991).

Compte tenu des difficultés à combattre les infections par mycobactéries, il existe un besoin urgent de pouvoir disposer d'une méthode rapide spécifique et sensible, permettant de diagnostiquer ces infections. Aussi, la détection précoce de *M. tuberculosis* dans des échantillons cliniques prend-elle une importance croissante dans le contrôle de la tuberculose tant pour le traitement clinique de patients infectés que pour l'identification d'individus à risque exposés.

La détection par PCR (Polymerase Chain Reaction) d'ADN spécifique d'espèces de mycobactéries est probablement l'une des nouvelles approches les plus prometteuses pour un diagnostic rapide, specifique et sensible (Saiki et al., 1985 ; Brisson-Noël et al., 1989; Cousins et al., 1992 ; Eisenach et al., 1990 ; Forbes et al., 1993 ; Fries et al., 1991 ; Hermans et al., 1990; Jonas et al., 1993 ; Kolk et al., 1992 ; Pierre et al., 1991 ; Saboor et al., 1992 ; Shankar et al.,1991 ; Sjöbring et al., 1990). Les différentes études réalisées jusqu'à ce jour ont toutefois abouti à des résultats différents en ce qui concerne la spécificité et la sensibilité. Parmi les raisons de cette diversité, on peut notamment citer des différences méthodologiques concernant la préparation des échantillons, le protocole suivi pour l'amplification ou les méthodes de détection des produits de PCR.

Plusieurs de ces études sont relatives à la détection par PCR du complexe *M. tuberculosis* à partir de l'ADN cible IS6110 (Clarridge et al., 1993 ; Eisenach et al., *1990*) ; Forbes et al., *1993*) *;* Noordhoeck et al., 1994), le gène codant pour l'antigène 65 kDa (Brisson-Noël et al., 1991 ; Telenti et al., 1993), le gène codant pour l'antigène 38 kDa (Folgueira et al., 1993 ; Sjöbring et al., 1990) ou l'ARN ribosomique 16S (Kox et al., 1995). Toutefois, tous ces tests de diagnostic identifient le complexe *M. tuberculosis* dans son ensemble.

Le complexe *M. tuberculosis* comprend *M. tuberculosis*, *M. bovis*, *M. microti* et *M. africanum*. Ces quatre espèces ont de fortes homologies dans leur ADN (85 à 100 %) (Imeada, 1985) et possèdent plusieurs gènes totalement identiques. Cette homologie a limité l'utilisation de séquences d'ADN pour différencier les souches. Il serait pourtant d'un intérêt particulier de pouvoir différencier les souches de *M*. *tuberculosis* de celles du bacille de Calmette-Guérin (BCG) *M. bovis,* ces dernières étant souvent utilisées comme vaccins vivants pour une immunoprotection contre la tuberculose. De façon évidente, il y a donc un intérêt à distinguer entre des mycobactéries susceptibles d'être pathogènes et des souches BCG vaccinantes. Cette distinction est particulièrement importante dans le cas d'individus immunodéprimés, tels que les sujets infectés par le VIH.

Les analyses du polymorphisme en longueur des fragments de restriction (RFLP ou Restriction Fragment Length Polymorphism) basées sur l'insertion de l'élément IS6110 ont été utilisées pour différencier différentes souches de *M tuberculosis*. On a montré que cet élément d'insertion permettait d'obtenir des profils de RLFP spécifiques de souches du fait d'une variabilité dans sa localisation et dans le nombre de copies existant dans les génomes de différentes souches. IS6110 a été mis en évidence chez *M tuberculosis* et chez *M. bovis* mais pas chez les autres mycobactéries testées (Cave et al., 1991). En général, plusieurs copies de cet élément d'insertion peuvent être mises en évidence chez *M. tuberculosis*, alors qu'une seule copie est trouvée chez *M. bovis* BCG (Cave et al., 1991). Toutefois, certaines souches de *M. tuberculosis* sont dépourvues de IS6110 (van Soolingen et al., 1994) et des souches de *M*. *bovis* possédant un nombre important de copies sont communes chez certaines populations (van Soolingen et al., 1994). S'ajoute à ces limitations le fait que l'identification des souches de *M. tuberculosis* à partir de IS6110 nécessite des analyses par Southern Blot et ne peut pas facilement être réalisée par des méthodes de PCR en routine.

Les auteurs de la présente invention ont mis en évidence une nouvelle séquence d'ADN cible pour amplification enzymatique. Cette séquence fait partie d'un opéron codant pour un système régulateur à deux composants spécifique de *M. leprae* et des bactéries membres du complexe *M. tuberculosis*. Les systèmes à deux composants sont des systèmes régulateurs appartenant à une grande famille, impliquant deux protéines qui coopèrent en traduisant des signaux externes par des modifications au niveau de l'expression génétique (*Parkinson et Kofoid, 1992.* Selon un modèle général proposé, un composant localisé dans la membrane agirait comme un capteur des changements environnementaux et transmettrait l'information à un composant régulateur de la réponse dans le cytoplasme, qui à son tour modulerait la transcription des gènes cibles. La communication entre les deux composants est généralement réalisée par une cascade de phosphorylations.

Les auteurs de la présente invention ont à présent cloné et caractérisé un système à deux composants mycobactériens. Ce système apparaît être spécifique des membres du complexe *M. tuberculosis* et de *M. leprae.* De façon inattendue par rapport aux autres systèmes à deux composants, les gènes de ce système mycobactérien sont séparés par des séquences d'ADN (séquences intercistroniques ou séquences répétées) uniquement présentes parmi les souches de mycobactéries du complexe *M. tuberculosis* et chez *M. leprae*.

Les auteurs de la présente invention ont par ailleurs montré que chez les souches du complexe *M. tuberculosis*, cette région intercistronique correspondait à un nombre de répétitions exactes ou tronquées d'une séquence de 77 paires de bases, SEQ ID N° 1, qui pouvait varier selon les souches. La séquence tronquée (SEQ ID N° 2) est composée de 53 paires de bases et contient une courte délétion interne en phase des nucléotides n° 40 à 66, qui sont substitués par un codon GAG.

Les auteurs de l'invention ont également montré que, de façon surprenante, la séquence tronquée (SEQ ID N° 2) était caractéristique des membres du complexe *M. tuberculosis* différents du BCG.

Chez *M. leprae,* la région intercistronique correspondante est constituée par un variant de 52 paires de bases déjà décrit par ailleurs et dont la séquence est indiquée ci-après : 5' atg aca ccc gcg cag gcg atg atg cag agc gaa gtg acg aga ggg aat gtg a 3'.

Compte tenu de leurs caractéristiques, ces séquences répétées offrent un intérêt particulier pour identifier et différencier entre elles des souches du complexe *M. tuberculosis* par des techniques d'amplification enzymatique, telles que la PCR ou autres méthodes similaires. Plus particulièrement, elles permettent d'établir un diagnostic différentiel entre la présence du BCG et celle d'autres membres du complexe dans un échantillon biologique. Ce diagnostic différentiel, dont le principe est basé sur la détection spécifique de la séquence SEQ ID N° 2, constitue un aspect préféré de l'invention. Il est avantageusement mis en oeuvre pour distinguer une infection par le BCG d'une infection par d'autres membres virulents du complexe chez des individus immunodéprimés, tels que notamment les sujets infectés par le VIH.

L'invention a donc pour objet un fragment d'acides nucléiques spécifique de mycobactéries appartenant au complexe *M. tuberculosis,* comprenant un enchainement de nucléotides choisi parmi la séquence SEQ ID N° 1, la séquence SEQ ID N° 2, leurs séquences complémentaires ou les séquences d'acides nucléiques capables de s'hybrider à l'une des séquences précédentes dans des conditions de forte stringence.

Elle vise également l'utilisation desdites séquences pour la fabrication de sondes nucléotidiques pour la détection in vitro de mycobactéries appartenant au complexe *M. tuberculosis* et d'amorces oligonucléotidiques pour l'amplification enzymatique de séquences spécifiques des souches membres du complexe *M. tuberculosis.*

L'invention a également pour objet une méthode permettant de différencier les souches du complexe *M. tuberculosis* entre elles, particulièrement de différencier le BCG des mycobactéries pathogènes de ce complexe.

Elle fournit également des moyens pour différencier des sous-groupes parmi les souches constituant le complexe *M. tuberculosis.*

Par ailleurs, elle peut permettre de différencier *M. leprae* des espèces et des souches appartenant au complexe *M. tuberculosis* dans un échantillon biologique.

Dans le cadre de la présente invention, on considère que les conditions de "forte stringence" dans lesquelles deux séquences nucléotidiques peuvent s'hybrider sont les conditions définies par Sambrook et al., 1989, à savoir des conditions de température comprises entre (Tₘ moins 5°C) et (Tₘ moins 30°C) et de préférence encore, des conditions de température comprises entre (Tₘ moins 5°C) et(Tₘ moins 10°C) (forte stringence), Tₘ étant la température de fusion, définie comme étant la température à laquelle 50 % des brins appariés se séparent. De manière préférentielle, les conditions de forte stringence utilisées correspondent à des températures de préhybridation, d'hybridation et de lavage de 68°C et un tampon de préhybridation et d'hybridation à base de 5 x SSC (protocole recommandé par Boehringer Mannheim).

L'invention a pour objet un fragment d'acides nucléiques spécifique de mycobactéries du complexe *M. tuberculosis* localisé dans la région intercistronique du système *sen*X3-*reg*X3.

Comme il sera expliqué dans les exemples, la séquence SEQ ID N° 1 correspond à une entité de 77 paires de bases répétée en un nombre différent de copies selon les souches de mycobactéries étudiées. Cette séquence répétée possède un cadre de lecture ouvert (ORF ou Open Reading Frame) pouvant coder pour un peptide de 25 acides aminés.

L'invention vise ainsi un fragment d'acides nucléiques spécifique du complexe *M. tuberculosis,* comprenant un enchainement de nucléotides choisi parmi la séquence SEQ ID N° 1, sa séquence complémentaire ou les séquences d'acides nucléiques capables de s'hybrider à l'une des séquences précédentes dans des conditions de forte stringence.

Selon un autre aspect, l'invention vise un fragment d'acides nucléiques spécifique de membres du complexe *M. tuberculosis* différents du BCG, notamment les espèces virulentes *M. tuberculosis*, *M*. *africanum ou M. bovis,* comprenant un enchaînement de nucléotides choisi parmi la séquence SEQ ID N° 2, sa séquence complémentaire ou les séquences d'acides nucléiques capables de s'hybrider à l'une des séquences précédentes dans des conditions de forte stringence.

Comme cela est en effet illustré par les exemples, les souches de BCG sont différentiables de toutes les autres souches du complexe *M. tuberculosis* à cause de l'absence de la séquence SEQ ID n° 2 dans la région intergénique *senX3-regX3* chez le BCG (voir tableau 3, groupes 4, 6, 8).

Les différentes séquences nucléotidiques de l'invention peuvent être d'origine artificielle ou non. Il peut s'agir de séquences d'ADN ou d'ARN.

Elles peuvent par exemple, être préparées par synthèse chimique, ou encore par des méthodes mixtes incluant des modifications chimiques ou enzymatiques de séquences obtenues par criblage de banques de séquences, au moyen de sondes élaborées sur la base des séquences SEQ ID N° 1 ou 2. De telles banques peuvent être préparées par des techniques classiques de biologie moléculaire, connues de l'homme de l'art.

Les séquences nucléotidiques de l'invention permettent la réalisation de sondes ou d'amorces nucléotidiques, capables de s'hybrider spécifiquement avec celles-ci, leurs séquences d'ARN correspondantes ou les gènes correspondants dans des conditions de forte stringence. De telles sondes font également partie de l'invention. Elles peuvent être utilisées comme outil de diagnostic in vitro pour la détection, par des expériences d'hybridation, de séquences d'acides nucléiques spécifiques de mycobactéries appartenant au complexe *M. tuberculosis*.

Préférentiellement, les sondes de l'invention comportent au moins 24 nucléotides consécutifs bien que des sondes plus courtes puissent également convenir. Au maximum celles-ci comportent la région intergénique *senX3-regX3* complète de *M. tuberculosis* (IPL), à savoir deux séquences SEQ ID N° 1 successives suivies d'une séquence SEQ ID N° 2. Ce fragment d'ADN comporte 218 paires de bases.

Parmi les sondes nucléotidiques préférées spécifiques des membres du complexe *M. tuberculosis*, figurent notamment la séquence SEQ ID N° 1 dans son intégralité ou sa séquence complémentaire.

Selon un autre aspect, l'invention vise des sondes nucléotidiques pour la détection et la mise en évidence de séquences d'acides nucléiques spécifiques de membres du complexe *M. tuberculosis* différents du BCG, notamment les espèces virulentes *M*. *tuberculoses, M. africanum* ou *M. bovis,* ainsi que pour le diagnostic différentiel de la présence du BCG dans un échantillon biologique contenant des mycobactéries du complexe *M. tuberculosis*.

De telles sondes nucléotidiques sont obtenues à partir d'une région de la séquence SEQ ID N° 2 encadrant le codon GAG à la position 40-42 ou de son brin complémentaire. Préférentiellement, cette région a une longueur de 21 paires de bases, et comporte 9 nucléotides en amont et en aval du codon GAG spécifique de la séquence SEQ ID N° 2.

Avantageusement, ces sondes comprennent la séquence SEQ ID N° 2 dans son intégralité ou sa séquence complémentaire.

Les sondes de l'invention hydrident selon les conditions d'hybridation appropriées, correspondant aux conditions de température et de force ionique usuellement utilisées par l'homme du métier.

Préférentiellement, les sondes de l'invention sont marquées, préalablement à leur utilisation. Pour cela, plusieurs techniques sont à la portée de l'homme du métier (marquage fluorescent, radioactif, chimioluminescent, enzymatique, etc).

Selon un mode de réalisation préféré, les sondes sont marquées à la digoxigénine. La digoxigénine (DIG) est un haptène stéroide couplé au dUTP pour marquer l'ADN utilisé comme sonde. Cette technologie est commercialisée par Boehringer Mannheim. Après l'hydridation avec la sonde et lavage, la détection se réalise suite à une émission d'un signal chemoluminescent grâce au substrat disodium 3-(4-methoxyspiro{1,2-dioxethane-3,2'-(5'-chloro)tricyclo decan}-4-yl)phenylphosphate (CSPD).

Les séquences nucléotidiques de l'invention sont également utiles pour la fabrication et l'utilisation d'amorces oligonucléotidiques pour des réactions de séquençage ou d'amplification enzymatique.

Les techniques d'amplification enzymatique sont principalement illustrées par la PCR. D'autres méthodes similaires peuvent toutefois être utilisées comme par exemple la LCR (Ligase Chain Reaction), la NASBA (Nucleic Acid Sequence Based Amplification), la Q-βréplicase, la SDA (Strand Displacement Amplification) et toute autre variante comprise dans les connaissances technologiques de l'homme du métier. Ces techniques d'amplification d'acides nucléiques utilisent des molécules d'amorces oligonucléotidiques pour initier la réaction d'élongation de la séquence cible. La longueur exacte de ces amorces pourra varier selon les cas. Par exemple, en fonction de la complexité de la séquence de la matrice, une amorce polynucléotidique contient typiquement de 15 à 25 nucléotides ou davantage. Dans certains cas, toutefois, elle peut en contenir moins.

Préférentiellement, les amorces nucléotidiques de l'invention comprennent au moins 19 nucléotides. Elles sont constituées par des amorces choisies sur des séquences adjacentes à la région intergénique *senX3-regX3,* dans les régions 3' de *senX3* et 5' de *regX3*.

Selon une variante préférée, on utilise le couple d'amorces appelées C5 et C3, à savoir 5'GCGCGAGAGCCCGAACTGC3' et 5'GCGCAGCAGAAACGTCAGC3'correspondant respectivement à l'extrémité 3' du gène *senX3* et l'extrémité 5' du gène *regX3*. Ces amorces s'hydrident respectivement 56 paires de bases en amont de la région intercistonique et 62 paires de bases en aval de celle-ci.

Les séquences nucléotidiques et les sondes en résultant peuvent être clonées dans des vecteurs de clonage et/ou d'expression selon des techniques de biologie moléculaire classiques, impliquant notamment l'utilisation d'enzymes de restriction et de sites de coupure spécifiques.

Un vecteur de clonage préféré dans la présente invention est représenté par le plasmide pRegXMt1 déposé à la CNCM sous le numéro I-1766 dont la construction est décrite plus en détail dans les exemples ci-après.

Un autre vecteur de clonage selon l'invention est représenté par le plasmide pRegX3Bc1 déposé à la CNCM sous le numéro I-1765. Ces plasmides ont été chacun introduits dans la bactérie *E. coli* XL1-blue.

Les vecteurs I-1765 et I-1766 contiennent chacun les gènes complets *senX3-regX3* avec les régions intercistroniques de BCG et de *M. tuberculosis* respectivement.

Les séquences nucléotidiques selon l'invention peuvent par ailleurs être exprimées dans des systèmes apropriés, pour la production et l'étude des actvités biologiques des peptides correspondants. Dans ce cas, celles-ci seront placées sous le contrôle de signaux permettant leur expression dans un hôte cellulaire.

Un système efficace de production d'une protéine ou d'un peptide recombinant nécessite de disposer d'un vecteur, par exemple d'origine plasmidique ou virale, et d'une cellule hôte compatible.

L'hôte cellulaire peut être choisi parmi des systèmes procaryotes, comme les bactéries, ou eucaryotes, comme par exemple les levures, cellules d'insectes, CHO (cellules d'ovaires de hamster chinois) ou tout autre système avantageusement disponible.

Le vecteur doit comporter un promoteur, des signaux d'initiation et de terminaison de la traduction, ainsi que les régions appropriées de régulation de la transcription. Il doit pouvoir être maintenu de façon stable dans la cellule et peut éventuellement posséder des signaux particuliers spécifiant la sécrétion de la protéine traduite.

Ces différents signaux de contrôle sont choisis en fonction de l'hôte cellulaire utilisé. A cet effet, les séquences nucléotidiques selon l'invention peuvent être insérées dans des vecteurs à réplication autonome au sein de l'hôte choisi, ou des vecteurs intégratifs de l'hôte choisi. De tels vecteurs seront préparés selon les méthodes couramment utilisées par l'homme du métier, et les clones en résultant peuvent être introduits dans un hôte approprié par des méthodes standard, telles que par exemple l'électroporation.

Les méthodes de diagnostic ou de détection in vitro dans lesquelles les sondes nucléotidiques obtenues à partir des séquences de l'invention sont mises en oeuvre, font également partie de la présente invention.

Plus particulièrement, l'invention vise une méthode de détection de souches de mycobactéries appartenant au complexe *M. tuberculosis* dans un échantillon biologique comprenant les étapes suivantes:
(i) mise en contact de l'échantillon biologique avec un couple d'amorces dans des conditions permettant une hybridation desdites amorces aux acides nucléiques spécifiques de souches de mycobactéries appartenant au complexe *M. tuberculosis ;*
(ii) amplification desdits acides nucléiques;
(iii) mise en contact d'une sonde nucléotidique selon l'invention avec ledit échantillon biologique dans des conditions permettant la formation de complexes d'hybridation entre ladite sonde et les séquences d'acides nucléiques amplifiés ;
(iv) détection des complexes d'hybridation formés.

Selon une première variante, la méthode selon l'invention permet de détecter la présence d'un membre quelconque du complexe *M*. *tuberculosis* dans un échantillon biologique. Dans ce cas, les complexes de l'étape (iii) définie ci-dessus sont formés avec une sonde nucléotidique spécifique de la séquence SEQ ID N° 1 ou de son brin complémentaire.

Selon une seconde variante avantageuse, la méthode de l'invention permet de spécifiquement détecter la présence des membres du complexe *M. tuberculosis* autres que le BCG. Selon cette variante, les complexes de l'étape (iii) qui sont détectés sont formés avec une sonde nucléotidique spécifique de la séquence SEQ ID N° 2 ou de son brin complémentaire, consistant préférentiellement en une séquence courte composée de deux fois 9 paires de bases encadrant le codon GAG aux positions 40 à 42 spécifique de la séquence SEQ ID N° 2.

Selon une troisième variante particulièrement avantageuse, la méthode de l'invention permet un diagnostic différentiel du BCG et des autres membres du complexe. Dans ce cas, la méthode consiste dans un premier temps en une mise en évidence d'acides nucléiques spécifiques de tous les membres du complexe *M. tuberculosis* par détection selon l'étape (iv) des complexes d'hybridation formés avec une première sonde nucléotidique spécifique de la séquence SEQ ID N° 1 ou de son brin complémentaire, puis à rechercher parmi les acides nucléiques amplifiés capables de former des complexes avec ladite première sonde, ceux qui peuvent également former des complexes avec une seconde sonde nucléotidique spécifique de SEQ ID N° 2 ou de son brin complémentaire.

Les séquences d'acides nucléiques amplifiés hydridant uniquement avec la première sonde correspondent à des séquences d'acides nucléiques spécifiques du BCG, alors que les séquences hydridant avec chacune des deux sondes correspondent à des séquences des autres membres du complexe *M. tuberculosis*.

Cette méthode de diagnostic différentiel offre un intérêt évident par rapport aux méthodes de détection classiques, car elle permet de différencier une infection par du BCG (issue éventuellement d'une vaccination), de celle par une mycobactérie virulente du complexe *M. tuberculosis (M. tuberculosis, M. bovis* ou *M. africanum*, et éventuellement *M. microti)*. Cette distinction est particulièrement importante chez des individus immunodéprimés, notamment des sujets infectés par le VIH.

Selon un autre mode de réalisation préféré, l'invention fournit une méthode d'identification de groupes de mycobactéries appartenant au complexe *M. tuberculosis*, caractérisée en ce que :
- on met en contact l'ADN desdites souches préalablement extrait avec un couple d'amorces telles que définies précédemment, dans des conditions permettant une hybridation spécifique desdites amorces à leurs séquences correspondantes sur l'ADN desdites souches et l'obtention de produits d'amplification, et
- on mesure la longueur des produits d'amplification obtenus, par exemple par électrophorèse en gel d'agarose.

Avantageusement, on utilise dans cette méthode les amorces 5'GCGCGAGAGCCCGAACTGC3' et 5'GCGCAGCAGAAACGTCAGC3'.

L'invention concerne également une trousse pour l'identification in vitro de souches de mycobactéries appartenant au complexe *M. tuberculosis* dans un prélèvement biologique comprenant :
- un couple d'amorces selon l'invention, comme définies précédemment ;
- les réactifs nécessaires pour permettre l'amplification des séquences d'acides nucléiques spécifiques de souches appartenant au complexe M. tuberculosis à l'aide desdites amorces,
- éventuellement des moyens pour révéler les fragments amplifiés, préférentiellement une sonde nucléotidique de l'invention.

D'autres caractéristiques et avantages de l'invention sont illustrés par les exemples dans la suite de la description ainsi que par les figures dont les légendes sont indiquées ci-après.

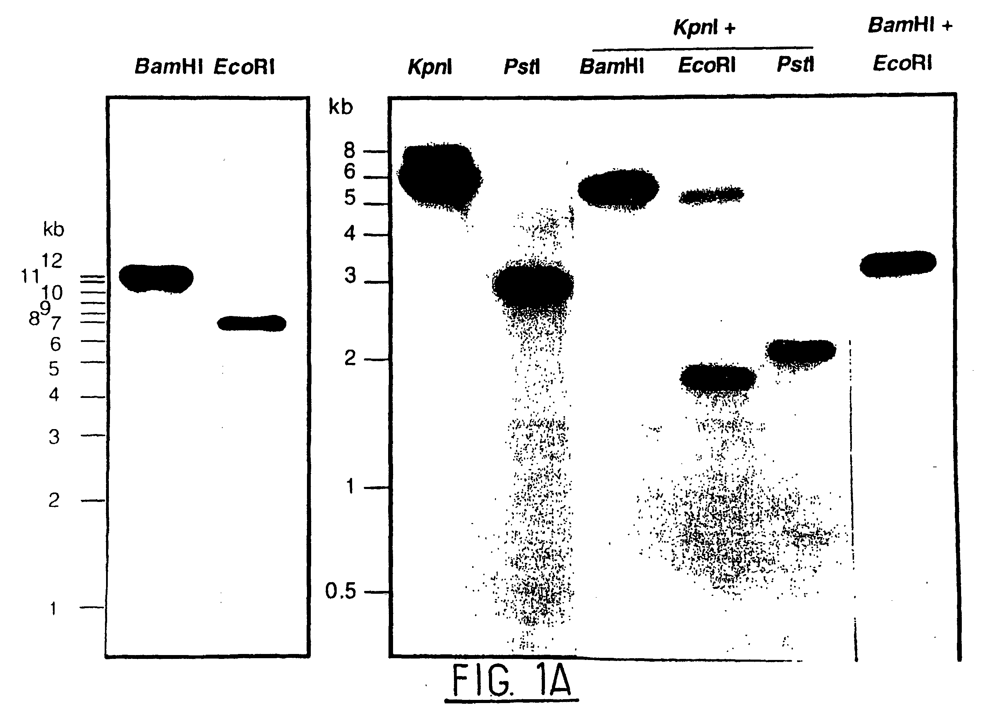

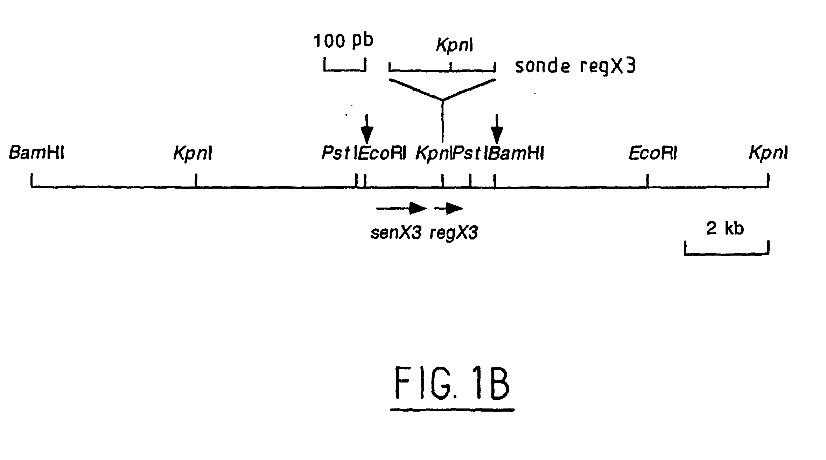

Figure 1 : Analyse par "Southern blot" de l'ADN de BCG (IPP). A). Le fragment de 259 paires de bases comprenant une partie du gène *regX3* a été utilisé comme sonde pour détecter le gène dans différents fragments de restriction dans l'ADN chromosomique du BCG(IPP). Les enzymes de restrictions utilisés sont indiqués en haut de la figure.
   B). Carte de restriction partielle du locus des gènes *senX3* et *regX3* du BCG (IPP). Les sites de restrictions sont indiqués par rapport aux gènes *senX3* et *regX3,* ainsi que la sonde utilisée.

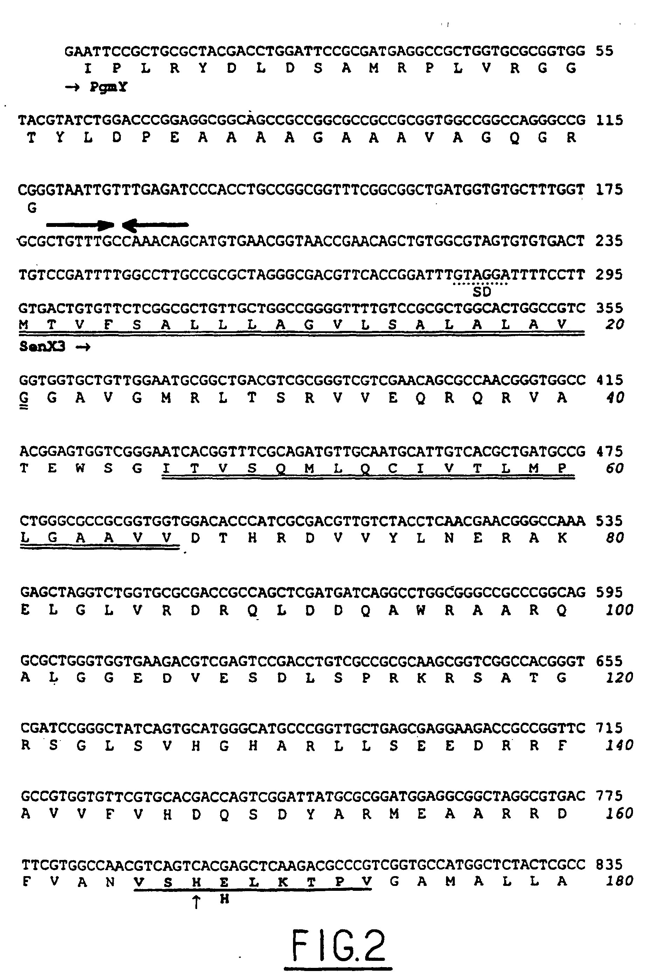

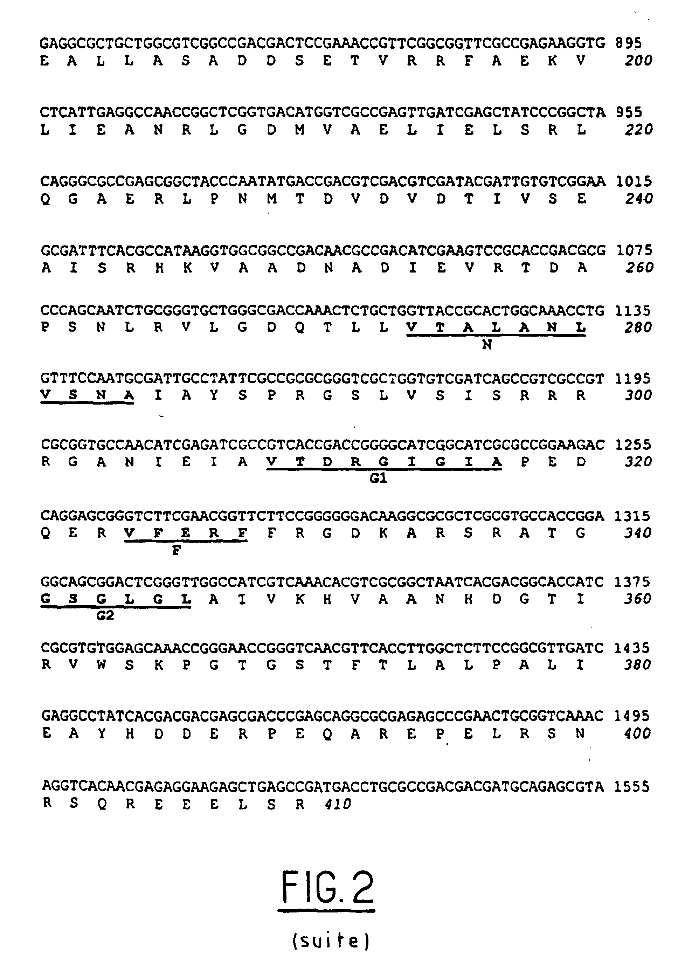

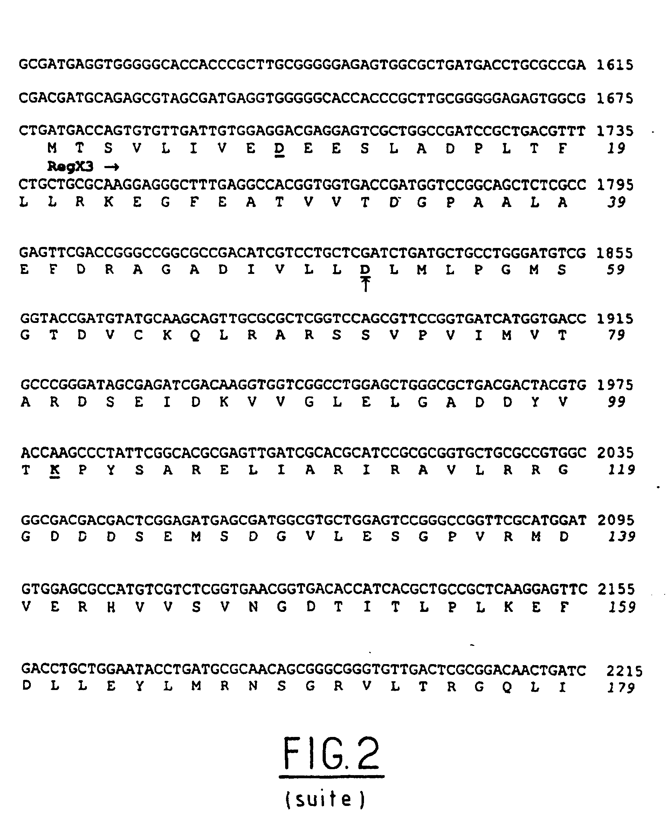

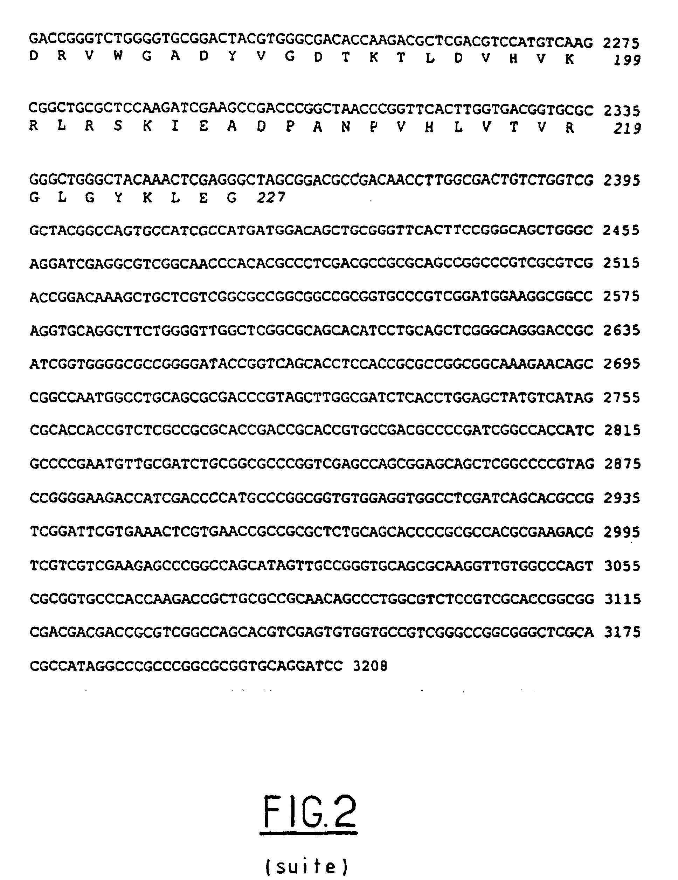

Figure 2 : Séquence nucléotidique des gènes *senX3* et *regX3* du BCG (IPP) et séquences protéiques dérivés. Les flèches indiquent des séquences palindromiques. La séquence Shin-Dalgarno putative est soulignée en pointillés (SD). La séquence transmembranaire prédite de SenX3 est doublement soulignée. Les régions de SenX3 conservées chez d'autres senseurs sont soulignées et annotées : H, région contenant l'histidine modifiée; N, région riche en asparagine; F, région riche en phénylalanine; G1 et G2, régions riches en glycine. Les petites flèches verticales indiquent les résidus prédits pour être phosphorylés. La séquence annotée PgmY indique la fin du gène codant la phosphoglycerate mutase.

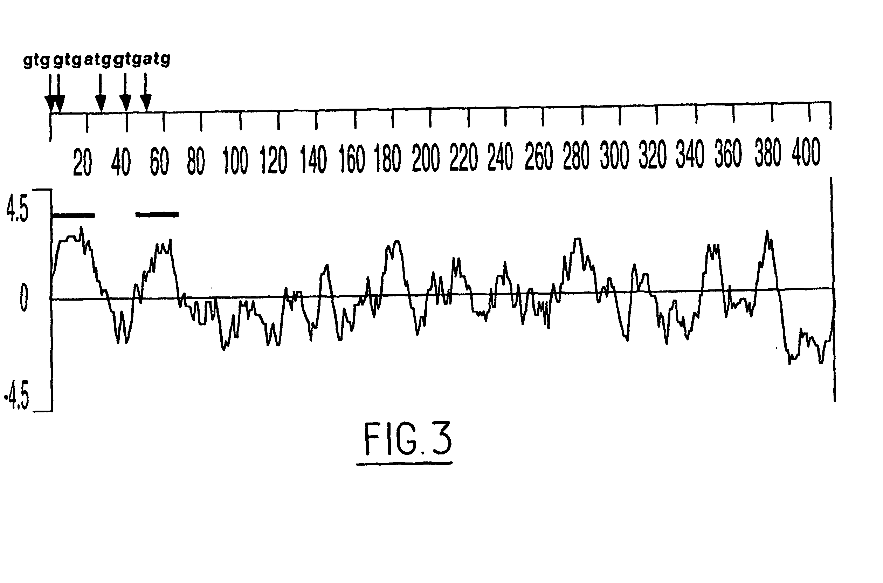

Figure 3 : Profil d'hydrophobicité de SenX3. Les valeurs positives indiquent des régions hydrophobes et les valeurs négatives indiquent les régions hydrophiles. Les flèches indiquent les codons d'initiation possibles et les deux lignes horizontales indiquent les régions transmembranaires prédites. Les chiffres au haut de la figure indiquent les numéros des acides aminés.

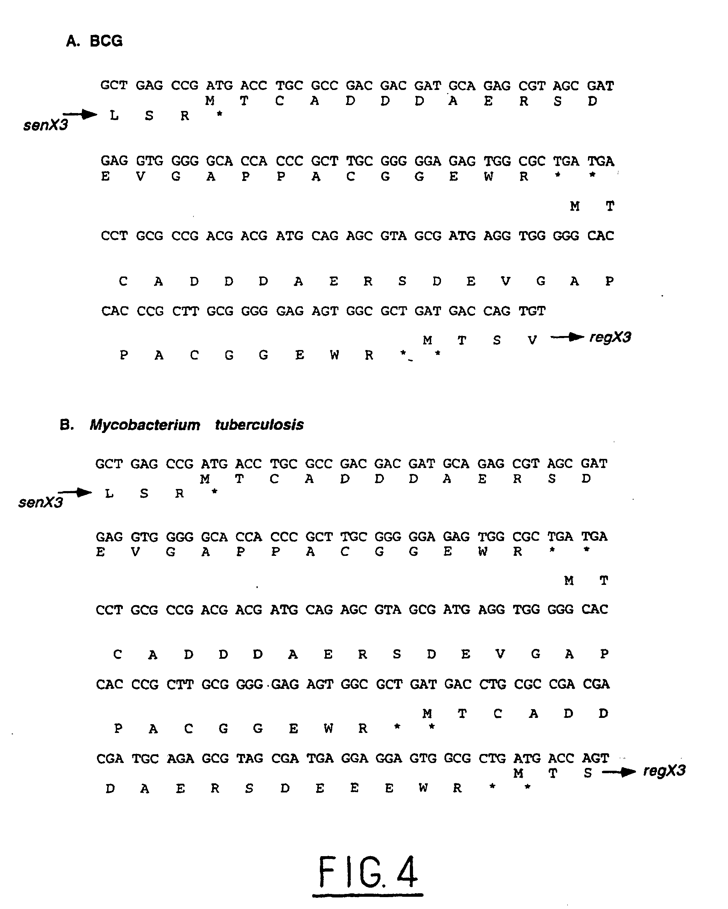

Figure 4 : Comparaison de la région intercistronique entre le BCG (IPP) et *M. tuberculosis* (IPL). Les gènes *senX3* et *regX3* sont indiqués par les flèches. Les séquences nucléotidiques sont indiquées ainsi que les séquences protéiques déduites.

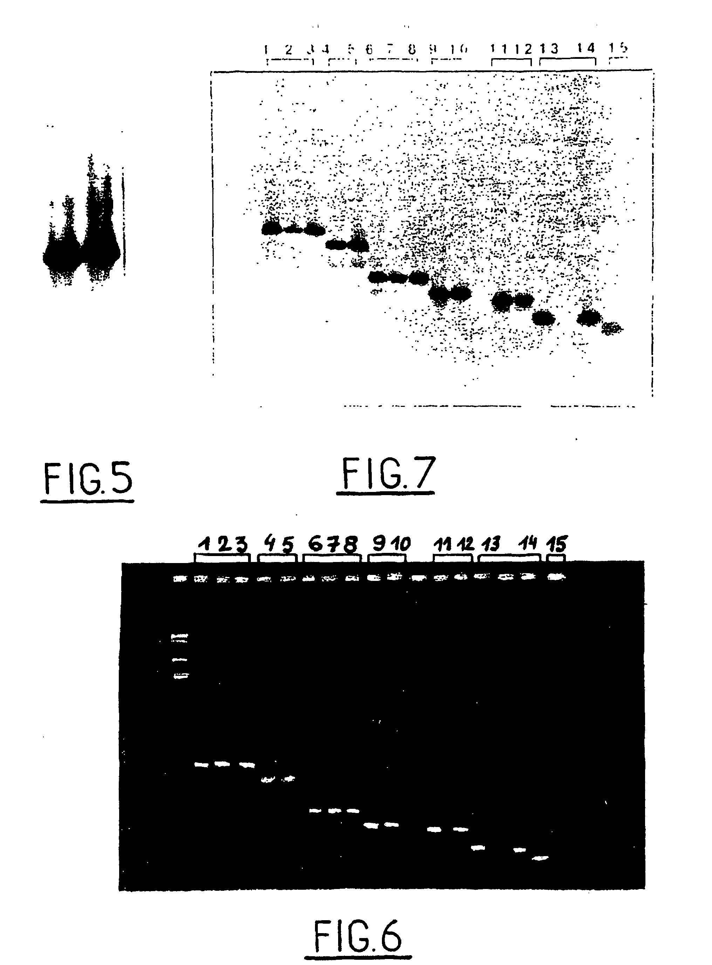

Figure 5 : Analyse par Southern blot d'ADN de *M. tuberculosis* et de BCG. L'ADN chromosomique de *M. tuberculosis* (IPL) (à droite) et de BCG (IPP) (à gauche) a été digéré par PstI, soumis à électrophorèse en agarose et analyse par hybridation avec la sonde intergénique *senX3-regX3* de *M. tuberculosis* (IPL).
Figure 6 : Analyse par électrophorèse en agarose (2,5%) des produits obtenus par PCR réalisée sur différentes souches mycobactériennes. Pistes 1 à 3 : groupe 1, respectivement *M. microti*, *M. tuberculosis V808, M. tuberculosis v761;* pistes 4 et 5, groupe 2, respectivement *M. tuberculosis V729, M. bovis* 60; pistes 6 à 8, groupe 3, respectivement *M. bovis 63, M. bovis* 78 et *M. bovis* AN5; pistes 9 et 10, groupe 4, respectivement *M. tuberculosis* H37Ra (IPL) et *M. tuberculosis* H37Rv (IPP); pistes 11 et 12, groupe 5, respectivement *M. tuberculosis* (IPL), *M. bovis* 76; pistes 13 et 14, groupe 6, respectivement *M. bovis* (BCGite 29) et *M. bovis* BCG (IPP); piste 15, groupe 7, *M. tuberculosis* N° 19.
Figure 7 : Analyse par Southern blot des fragments obtenus dans la figure 6. La sonde utilisée est la région intercistronique *senX3-regX3* de *M. tuberculosis* (IPL).

### EXEMPLES

### EXEMPLE 1 : Carte génomique et clonage des gènes senX3-regX3 de M.bovis BCG (IPP), codant pour le système à deux composants mycobactérien.

Wren et al. (1992) ont amplifié un fragment de 259 paires de base provenant du gène de *M. tuberculosis* (IPL) qu'ils ont appelé *reg*X3.

Les auteurs de la présente invention ont amplifié la séquence correspondante à partir de *M. bovis* BCG (souche vaccinale 1173P2, obtenue à la collection WHO de Stockholm, Suède) en utilisant les oligonucléotides synthétiques suivants : 5'-CGAGGAGTCGCTGGCCGATCCGC-3' - et 5'-AGCGCCCCAGCTCCAGCCGACC-3'. L'amplification a été réalisée en utilisant ces amorces et une polymérase Deep Vent (New England Biolabs). Le produit d'amplification de 259 paires de base résultant a ensuite été purifié par électrophorèse sur gel d'agarose puis sous-cloné dans le site *Sma*I du vecteur pBluescript KS+ (Stratagene) selon des protocoles standards (Sambrook et al., 1989). Cet insert a ensuite été retiré du plasmide recombinant par digestion avec *Bam*HI et *Eco*RI puis marqué avec du dCTP[α-³²P] par "random priming" (marquage au hasard) en utilisant le kit de "random priming" commercialisé par Boehringer selon les conditions recommandées par le fabricant. L'ADN génomique de *M. bovis* BCG (IPP), mis en culture dans du milieu de Sauton (Sauton, 1912) à 37°C dans des flacons pour culture stationnaire de tissus, a été extrait comme décrit auparavant (Kremer et al., 1995a) et digéré par différentes enzymes de restriction. Cet ADN a ensuite été soumis à une électrophorèse sur gel d'agarose et à une analyse par Southern Blot selon les méthodes standards (Sambrook et al., 1989). Le sondage de l'empreinte avec de l'ADN marqué au [³²P] a montré qu'une digestion par K*pn*I avec soit *Bam*HI, *Eco*RI ou P*st*I, produisait des paires de bandes hybridantes, avec une bande marquée de façon plus intense que l'autre dans chacune des paires (fig. Ia). Ces bandes ont été attribuées aux séquences 5' et 3' respectivement, du site KpnI asymétrique unique de la sonde. Ceci a permis de localiser les sites 5' et 3' *Bam*HI, *Eco*RI et P*st*I du site K*pn*I (fig. Ib). Les digestions avec uniquement P*st*I ont donné une seule bande d'hybridation. Comme cela pouvait être prévu, la digestion avec K*np*I a donné deux bandes et la digestion avec *Bam*HI et *Eco*RI a conduit à l'obtention d'une seule bande d'environ 3,5 kb.

L'ADN génomique du BCG a été digéré avec *Bam*HI et *Eco*RI, et des fragments d'ADN de 3 à 4 kb ont été isolés après une électrophorèse sur gel d'agarose. Ces fragments ont été insérés dans pBluescript SK-(Stratagene) restreint par *Bam*HI et *Eco*RI. 900 clones recombinants ont été criblés avec la sonde marquée au [³²P] par la technique standard dite de "colony blot hybridization" (Sambrook et al., 1989). Trois d'entre eux étaient positifs et se sont avérés contenir le même insert d'ADN de 3,2 kb *Bam*HI/*Eco*RI par analyse de restriction. Un clone hybridant a été isolé pour des études ultérieures et a été dénommé pRegX3Bc1 (I-1765) .

### EXEMPLE 2. : Séquence des gènes de M. bovis BCG (IPP) codant pour le système à deux composants

Les fragments *Sal*I de 1,0, 1,5 et 0,7 kb ont été isolés de l'insert de 3,2 kb de pRegX3Bc1 (I-1765), et sous- clonés dans pBluescript SK-. La séquence de l'insert de 3,2 kb, ainsi que celle des sous-clones SalI a été déterminée sur les deux brins par la méthode de "primer walking". La séquence nucléotidique de 3208 pb de *M. bovis* BCG (IPP) (Fig. 2) a démontré la fréquence de C + G caractéristique des mycobactéries (66,6 %). Deux principaux cadres de lecture ouverts (ORF, Open Reading Frame) désignés par *reg*X3 et *sen*X3, ont été identifiés (Fig. Ib et 2). Le gène *reg*X3 démarre par un triplet ATG à la position 1679 et se termine par un triplet TAG à la position 2360. Il contient la séquence de la sonde marquée au ³²P et code pour une protéine dont la séquence en acides aminés déduite est de 227 résidus pour une masse moléculaire calculée de 24 881 Da. La limite en amont de l'ORF *sen*X3 n'est pas certaine parce que cinq codons d'initiation potentiels en phase (ATG ou GTG) ont été trouvés sur une courte distance (de 296 à 446) (Fig. 2 et 3). Toutefois, seul le GTG à la position 296 est précédé à 9 nucléotides en amont, par une séquence homologue à la séquence de Shine-Dalgarno de *Escherichia coli* (4 des 6 résidus sont identiques à AGGAGG). Donc, ce codon GTG pourrait être la limite en 5' de l'ORF *senX3* qui se termine avec le codon stop TGA à la position 1526. Cet ORF est donc présumé coder pour une protéine de 44769 Da composée de 410 résidus d'acides aminés. Le gène *senX3* est précédé à 273 nucléotides en amont par la partie 3' d'un ORF codant un homologue des phosphoglycérate mutases. Une séquence susceptible de former une structure en épingle à cheveux est localisée entre les positions 178 et 194. Cette séquence pourrait fonctionner comme une séquence terminatrice de transcription de l'ORF en amont.

Une recherche sur banque de donnée MycDB a révélé que les gènes *sen*X3 et *reg*X3 sont aussi présents chez *M. leprae*. Les produits qu'ils codent sont similaires respectivement à 82,7 % et 94,9 % aux protéines senX3 et RegX3 de *M*. *bovis* (IPP). L'ORF *sen*X3 de *M. leprae* s'avère être initié par un codon GTG et est précédé par un ORF codant pour un homologue de phosphoglycérate mutase, similaire à celui qui a été trouvé chez *M. bovis* BCG (IPP).

### EXEMPLE 3 : Clonage et séquençage des gènes senX3-regX3 de M. tuberculosis (IPL).

Les gènes *sen*X3 et *reg*X3 de *M. tuberculosis* (IPL) ont été clonés par PCR à partir d'ADN chromosomique de *M. tuberculosis* 22962067, un isolat clinique de la collection de mycobactéries de l'Institut Pasteur de Lille. L'ADN de *M. tuberculosis* (IPL) a été extrait par la méthode décrite précédemment pour l'extraction d'ADN chromosomique du BCG (Kremer et al., 1995a). Le fragment de 2,2 kb contenant les gènes *sen*X3 et *reg*X3 de *M*. *tuberculosis* (IPL) a été amplifié par PCR en utilisant les amorces suivantes, homologues aux séquences adjacentes des gènes *sen*X3 et *Reg*X3 de *M*. *bovis* BCG (IPP): 5'-TGGCGTAGTGTGTGACTTGTC-3' et 5'-GACCAGACAGTCGCCAAGGTT-3'. Le fragment amplifié a été cloné dans le site *Sma*I de pBluescript SK- (version II) pour produire un plasmide dénommé pRegX3Mt1 (I-1766). Le fragment total de 2,2 kb a ensuite été séquencé en utilisant la même stratégie que celle décrite dans l'exemple 2 pour les gènes *sen*X3 et *reg*X3 du BCG (IPP). La séquence d'ADN des ORF *sen*X3 et *reg*X3 de *M*. *tuberculosis* (IPL) ainsi que la région 5' en amont de *sen*X3 et la région 3' en aval de *Reg*X3 étaient identiques à celles du BCG (IPP). Toutefois, la région intercistronique entre *sen*X3 et *Reg*X3 démontrait des différences intéressantes qui sont étudiées dans l'exemple ci-après.

### EXEMPLE 4 : Analyse de la région intercistronique senX3-regX3.

La région intercistronique entre *sen*X3 et *Reg*X3 contient une parfaite duplication de 77 paires de bases en tandem chez le BCG (IPP) (fig. 4). Chaque séquence répétée contient un court ORF qui a la capacité de coder pour un peptide de 25 acides aminés. Le codon d'initiation ATG que l'on peut déduire de la première répétition chevauche le codon stop TGA de *sen*X3. Cette séquence répétée se termine par deux codons stop en phase, qui chevauchent le codon d'initiation ATG déduit de la répétition suivante. L'ORF de la seconde répétition se termine aussi par un double codon stop TGA, qui chevauche le codon start ATG de *reg*X3.

La région intercistronique de *M. tuberculosis* (IPL) est plus longue et contient une troisième séquence répétée qui toutefois n'est pas complète. Elle contient une courte délétion interne en phase des nucléotides 40 à 66 qui sont remplacés par GAG. L'ORF de cette troisième répétition se termine aussi par un double codon stop chevauchant le codon ATG de *reg*X3.

La séquence de la région intercistronique de *M. leprae* est plus courte que celle du BCG (IPP). Elle contient 52 paires de bases.

L'existence de ces structures en répétition dans la région intercistronique *sen*X3-*reg*X3 a conduit les inventeurs à rechercher si elles étaient présentes dans d'autres régions de *M. tuberculosis* (IPL) ou dans le chromosome de *M. bovis* BCG (IPP) par analyse par Southern Blot. La région intercistronique *sen*X3-*reg*X3 de *M. tuberculosis* (IPL) a été obtenue par digestion enzymatique de pRegX3Mt2 avec *Eco*RI et *Bam*HI. Le fragment d'ADN de 491 paires de bases résultant a été ensuite digéré avec B*sr*I-*Alu*I pour produire un fragment d'ADN de 218 paires de bases qui correspond au fragment intergénique *sen*X3-*reg*X3 de *M. tuberculosis* (IPL). Ce fragment d'ADN a ensuite été marqué au hasard avec de la digoxigénine-dUTP (kit cat. N° 1093657) selon les recommandations du fabricant (Boehringer Mannheim). pRegX3Mt2 a été produit par une première amplification par PCR d'un fragment d'ADN chromosomique de 471 paires de bases de *M. tuberculosis* 22962067 (IPL) en utilisant les oligonucléotides suivants : 5'AAACACGTCGCGGCTAATCA 3' et 5'CCTCAAAGCCCCTCCTTGCGC 3' et le fragment amplifié résultant a ensuite été cloné dans le site *Sma*I de pBluescript KS-.

L'ADN chromosomique de *M. tuberculosis* (IPL) a ensuite été totalement digéré avec PstI et soumis à une électrophorèse sur gel d'agarose et analysé par Southern blot selon les procédures habituelles (Sambrook et al., 1989). La sonde marquée a ensuite été utilisée pour une hybridation comme indiqué ci-après.

La sonde a d'abord été dénaturée par ébullition pendant 5 minutes, et la membrane a été incubée avec la sonde pendant la nuit à 68°C, après une préhybridation pendant deux heures à 68°C. Le tampon utilisé pour la préhybridation et l'hybridation était du 5xSSC (Sambrook et al., 1989), 0,1% de N-laurylsarcosine (p/v) ; 0,02 % de SDS (p/v) ; 1 % de réactif bloquant (Boehringer Mannheim). La membrane a ensuite été lavée deux fois pendant cinq minutes dans du 2xSSC (Sambrook et al., 1989) et 0,1 % de SDS à température ambiante et deux fois pendant 15 minutes dans du 0,1xSSC, 0,1 % SDS à 68°C. Les sondes hybridées ont ensuite été immunodétectées avec des fragments Fab antidigoxigénine conjugués à la phosphatase alcaline et un substrat CSPD chimioluminescent (Boehringer Mannheim).

Comme on peut le voir sur la figure 5, différentes copies de la région intercistronique *sen*X3-*reg*X3 sont présentes chez *M. tuberculosis* (IPL), ainsi que chez *M. bovis* BCG (IPP).

### EXEMPLE 5 : Amplification par PCR de la région intercistronique senX3-regX3 de différentes. souches de mycobactéries.

Etant donné que la région intercistronique séparant les gènes *senX3* et *regX3* présente des variations de longueur entre *M. bovis* BCG (IPP), *M. tuberculosis* (IPL) et *M. leprae,* les auteurs de la présente invention ont analysé la région correspondante chez d'autres souches de *M. bovis* (y compris des souches non BCG) et de *M. tuberculosis*. Ils ont également analysé d'autres espèces de mycobactéries : (i) les autres membres du complexe *M. tuberculosis* : *M. africanum* et *M. microti* et (ii) les mycobactéries autres que celles du complexe *M. tuberculosis*.

Les souches analysées sont indiquées dans les tableaux 1 et 2. Leur ADN chromosomique a été préparé comme indiqué ci-après. Les mycobactéries ont été récupérées par centrifugation (3000 tours/min ; 30 minutes), à partir de 100 ml de culture et mises en incubation pendant 1 heure à 37°C dans 10 ml de tampon phosphate (saccharose 0,4 M, EDTA 10 mM, Tris-HCl (pH 8,) 10 mM, 4 mg/ml de lysozyme). Les protoplastes obtenus ont été récupérés par centrifugation (3000 tours/min ; 20 minutes) puis lysés en les mettant à incuber pendant 1 heure à 60°C dans 6 ml de tampon L (NaCl 10 mM, SDS 6 %, Tris-HCl (pH 8) 10 mM, 500 µg/ml de protéinase K). Après addition de 1,5 ml de NaCl 5 M, le mélange a été centrifugé (14 000 tours/min., 20 minutes). Le surnageant a été soumis à une extraction au phénol-chloroforme et l'ADN a été précipité à l'isopropanol. Le culot remis en suspension a ensuite été traité par la RNAse, extrait avec du phénol-chloroforme et du chloroforme puis précipité avec de l'éthanol. Le culot a été ensuite séché à l'air et remis en suspension dans 100 µl de tampon TE (10 mM Tris-HCl, 1mM EDTA, pH 7,6).

Les amorces pour analyse par PCR ont -été choisies à partir de la séquence d'ADN des gènes *sen*X3 et *Reg*X3 de *M. bovis* BCG (IPP) et *M. tuberculosis* (IPL). Une amorce (C5) hybridait à l'extrémité 3' du gène *sen*X3 et avait la séquence 5'-3' suivante : 5'GCGCGAGAGCCCGAACTGC3'. L'autre amorce (C3) hybridait à l'extrémité 5' du gène *Reg*X3 et avait la séquence 5'-3' suivante : 5'-GCGCAGCAGAAACGTCAGC-3'. Avec ces deux amorces, le produit de PCR comprend 56 paires de bases additionnelles à l'extrémité 5' de la région intergénique et 62 paires de bases additionnelles à son extrémité 3' par rapport à la région intercistronique. Le produit de PCR a une longueur de 369 paires de bases pour *M. tuberculosis* 22962067 (IPL) et 276 paires de bases pour *M. bovis* BCG (IPP).

Les amplifications par PCR ont été réalisées dans un termocycleur (Perkin Elmer) par incubation de 1 µl d'ADN chromosomique avec le mélange réactionnel suivant : oligonucléotides C5 et C3 (1 µg/1µl de chaque), dTNP (2 µl) 25 mM, TMACl (chlorure de tétraméthylammonium) (2 µl) 5 mM, tampon enzymatique 10 x (10 µl), ADN polymérase Vent (1 U/0,5 µl), eau (82 µl). L'amplification a été réalisée sur 30 cycles à 94°C pendant une minute, 65°C pendant une minute et 72°C pendant une minute. 10 µl de produit de PCR ont ensuite été soumis à une électrophorèse sur gel d'agarose à 2,5 % et visualisés avec du bromure d'éthidium. Les contrôles négatifs contenant tous les réactifs de PCR excepté la matrice d'ADN, ont été traités en parallèle avec les échantillons. La longueur du produit de PCR a été estimée par comparaison avec l'échelle d'ADN de 1 kb.

Aucun produit de PCR n'a été détecté pour les 11 souches de mycobactéries non tuberculosis testées. (voir tableau 2). Trois souches de *Streptomyces* (*S. cacaoi,* S. R61 et S. R39), ainsi que *E. coli* TG1 ont également donné des résultats négatifs en PCR. En revanche, des produits de PCR ont été obtenus avec tous les membres du complexe *M. tuberculosis*. La spécificité des produits de PCR a été confirmée dans tous les cas par analyse par Southern Blot en utilisant en tant que sonde la région intergénique *sen*X3-*reg*X3 de *M. tuberculosis* (IPL) marquée à la digoxigénine, comme décrit à l'exemple 4.

La longueur des fragments amplifiés est indiquée dans le tableau 3. Huit groupes différents avec des longueurs d'amplicon différentes ont été obtenus. sur les 35 isolats cliniques de *M. tuberculosis* testés, 34 ont donné des fragments de PCR de 329 paires de bases qui ne pouvaient être distingués de celui obtenu avec la souche référence *M. tuberculosis* 22962067 (IPL) (groupe 5). Une souche de *M. tuberculosis* (N° 19) a donné un produit de PCR de 254 paires de bases (groupe 8). *M. tuberculosis* S200 appartient aussi au groupe 5.

Des souches de *M. tuberculosis* provenant du Vietnam et ne contenant pas la séquence IS6110 (V. 808, V. 761 et V.729) étaient différentes du groupe majeur *M. tuberculosis* (groupe 5). La longueur de leur produit de PCR dépassait de 500 paires de bases (un produit de +/-500 paires de base et deux de 560). Les souches de laboratoire de *M. tuberculosis* H37Ra et H37Rv avaient des produits de PCR légèrement plus grands que ceux obtenus dans le groupe 5 des souches de *M. tuberculosis* et ces deux souches sont classées parmi le groupe 4. Ces deux souches sont particulières en ce qui concerne la pathogénicité incertaine pour H37Rv et la perte de pathogénicité de H37Ra. Par ailleurs, aucun cas clinique de *M. tuberculosis* n'était présent dans les groupes 4, 6, 8; ces trois groupes sont donc spécifiques pour les souches non-virulentes.

Les souches BCG (souches vaccinales et isolats de cas cliniques de BCGites) ont été divisées en trois groupes (N° 4, 6 et 8). Les produits de PCR obtenus à partir de ces souches avaient une longueur de 353 paires de base, 276 et 199, respectivement.

La variabilité la plus importante au niveau de la longueur des fragments de PCR était rencontrée pour les souches de *M. bovis* non BCG. Six souches ont été testées. Trois d'entre elles, y compris la souche référence AN5, ont donné des produits de PCR de 406 paires de bases (groupe 3). Les trois autres souches ont été regroupées dans les groupes 2, 5 et 7 correspondant à des fragments de PCR d'environ 500 et de 329 ou 254 paires de bases respectivement.

**Tableau 1 :**

| **Complexe *M. tuberculosis*** | | |
|---|---|---|
| Espèces | Souches | Sources |
| *M. tuberculosis* | - référence (22962067) | - IPL* |
| | - H37Ra | - IPP** |
| | - H37Rv | - IPP (Marchal) |
| | - S200 | - CHR de Lille*** |
| | - 35 isolats cliniques (différents profils de IS6110 RFLP) | - CHR de Lille |
| | - 3 isolats cliniques du Vietnam : V808, V761, V729 | - IPP (Marchal) |
| | | |
| *M. bovis* | - BCG (souches vaccinales): | - IPP |
| | BCG IPP | - IPL |
| | BCG Moreau | - IPL |
| | BCG Japonicus | - IPP |
| | BCG Pragues | - IPP |
| | BCG Montréal | - IPP |
| | BCG Russe | - IPP |
| | BCG Glaxo | |
| | - cas de BCGites : | |
| | 4 isolats cliniques (28, 29 30,31) | - IPL - CHR de Lille |
| | - non BCG : | |
| | référence ANS | - IPP (Marchal) |
| | 1 isolat clinique (1) | - CHR de Lille |
| | 2 isolats de chèvres (60, 63) | - Université de Zaragoza |
| | 2 isolats de vaches (76, 78) | (Martin) - Université de Zaragoza |
| | | |
| *M. africanum* | - isolat clinique | (Martin) |
| | | |
| *M. microti* | - référence ATCC 19422 | - CHR de Lille |
| | | - IPP (Marchal) |

| | | |
|---|---|---|
| * Institut Pasteur de Lille | | |
| ** Institut Pasteur de Paris | | |
| *** Centre Hospitalier Régional de Lille | | |

**Tableau 2 :**

| **Mycobactéries atypiques** | |
|---|---|
| Espèces et Souches | Sources |
| *M. aurum* | - isolats cliniques (IPL) |
| *M. avium* | - isolats cliniques (IPL) |
| *M. chelonae* | - isolats cliniques (IPL) |
| *M. flavescens* | - isolats cliniques (IPL) |
| *M. fortuitum* | - isolats cliniques (IPL) |
| *M. kansasii* | - isolats cliniques (CHR de Lille) |
| *M. marinum* | - isolats cliniques (IPL) |
| *M. scrofulaceum* | - isolats cliniques (IPL) |
| *M. smegmatis* | - IPL |
| *M. terae* | - isolats cliniques (IPL) |
| *M. xenopi* | - isolats cliniques (IPL) |

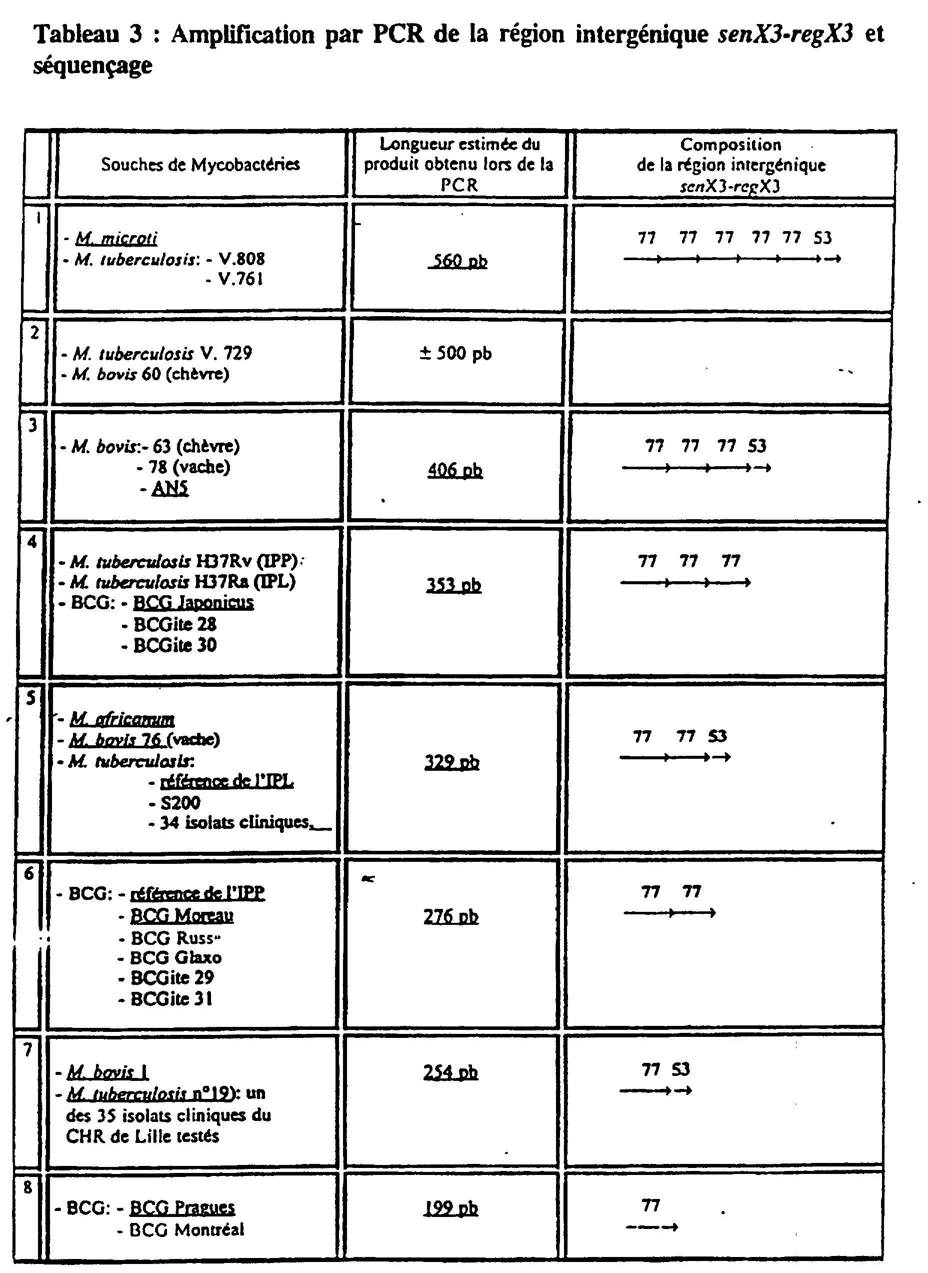

Les fragments d'ADN amplifiés comprennent la région intercistronique *senX3-regX3* ainsi que 56 paires de bases en amont de celle-ci et 62 paires de bases en aval. Les flèches 77 et 53 désignent les éléments répétés trouvés dans chaque groupe. Les souches pour lesquelles la région intercistronique a été séquencée sont soulignées.

### BIBLIOGRAPHIE

Barnes, P.F., A.B. Bloch, P.T. Davidson, and D.E. Snider. 1991. Tuberculosis In patients with human immunodeficiency virus infection. N. Engl. J. Med. 324: 1644-1650.
Brlsson-Noel, A., D. Lecossier, X. Nassif, B. Giquel, V. Levy-Frebault, and A.J. Hance. 1989. Rapid diagnosis of tuberculosis by amplification of mycobacteria DNA in clinical samples. Lancet ii: 1069-1072.
Brisson-Noel, A., C. Aznar, C. Chureau, S. Nguyen, C. Pierre, M. Bartoli, R. Bonete, G. Pialoux, B. Gicquel, and G. Garrigue. 1991. Diagnosis of tuberculosis by DNA amplification in clinical practice evaluation. Lancet 338: 364-366.
Cave, M.D., K.D. Eisenach, P.F. McDermott, J.H. Bates, and J.T. Crawford. 1991. IS6110: conservation of sequence in the Mycobacterium tuberculosis complex and its utilization in DNA fingerprinting. Mol. Cell. Probes 5: 73-80.
Clarridge, J.E., R.M. Shawar, T. Shinnick, and B.B. Plikaytis. 1993. Large-scale use of polymerase chain reaction for detection of Mycobacteria tuberculosis in a routine mycobacteriology laboratory. J. Clin. Microbiol. 31: 2049-2056.
Cousins, D.V., S.D. Wilton, B.R. Francis, and B.L. Beth. 1992. Use of PCR for rapid diagnosis of tuberculosis. J. Clin. Microbiol. 30: 255-258.
Bisenbach, K.D., D. Cave, J.II. Bates, and J.T. Crawford. 1990. Polymerase chain reaction amplification of a repetitive DNA sequence specific for Mycobacterium tuberculosis. J. Infect. Dis. 161: 977-981.
Folgueira, L., R. Delgado, E. Palenque, and A.R. Noriega. 1993. Detection of Mycobacterium tuberculosis DNA in clinical samples by using a simple lysis method and PCR. J. Clin. Microbiol. 31: 1019-1021.
Forbes, B.A. and K. Hicks. 1993. Direct detection of Mycobacteria tuberculosis in respiratory specimens in a clinical laboratory by PCR. J. Clin. Microbiol. 31: 1688-1694.
Fries, J.W.U., R.J. Patel, W.F. Piessens, and D.F. Wirth. 1991. Detection of untreated mycobacreia by using PCR and specific DNA probes. J. Clin. Microbiol. 29: 1744-1747.
Hermans, P.W.M., A. R.J. Schuitema, D. vam Soolingen, C.P.H.J. Vcrstynen, E.M. Blk, J.E.R. Thole, A.H.J. Kolk and J.D.A. van Embden. 1990. Specific detection of Mycobacterium tuberculosis complex strains by PCR. J. Clin. Microbiol. 28: 1204-1213
Imaeda, T. 1985. Deoxyribonucleic acid relatedness among selected strains of Mycobacterium tuberculosis, M. bovis, M. bovis BCG, M. microti, and M. africanum. Int. J. Syst. Bacteriol. 35: 147-150.
Jonas, J. Clin. Microbiol., M.J. Alden, and J.I. Curry. 1993. Detection and identification of Mycobacteria tuberculosis directly from sputum sediments by amplification of rRNA. J. Clin. Microbiol. 31: 2410-2416.
Kolk, A.H.I., A.R.J. Schuitema, S. Kuiper, V. van Leeuwen, P.W.M. Hermans, J.D.A. van Embden, and R.A. Hartskeerl. 1992. Detection of Mycobacterium tuberculosis in clinical samples by using PCR and a no,radioactive detection system. J. Clin. Microbiol. 30: 2567-2575.
Kox, L.F.F., J. van Leeuwen, S. Knijper, H.M. Jansen, and A.H.J. Kolk. 1995. PCR assay based on DNA coding for 16S rRNA for detection and identification of mycobacteria in clinical samples. J. Clin. Microbiol. 33: 3225-3233.
Kremer, L., A. Baulard, J. Estaquier, O. Poulain-Godefroy, and C. Locht. 1995a. Green fluorescent protein as a new expression marker in mycobacteria. Mol. Microbiol. 17: 913-922.
Kremer, L., A. Baulard, J. Estaquier, J. Content, A. Capron, and C. Locht. 1995b. Analysis of the Mycobacterium tuberculosis 85A antigen promoter region. J. Bacteriol. 177:642-653.
Noordhoek, G.T., A.H.J. Kolk, G. Bjune, D. Catty, J.W. Dale, P.E. Fine, P. Godfrey-Paussett, S.-N. Cho, T. Shinnick, S.B. Svenson, S. Wilson, and J.D.A. van Embden. 1994. Sensitivity and specificity of PCR for detection of Mycobacterium tuberculosis: a blind comparison study among seven laboratories. J. Clin. MicrobioL 32: 277-284.
Parkinson, J.S. and Kofoid, E.C. 1992 Communication modules in bacterial signaling proteins. Annu. Rev. Genet. 26, 71-112
Pierre, C., D. Lecossier, Y. Boussougnant, D. Bocart, V. Joly, P. Veni. and A. Hance. 1991. Use of a reamplifiaction protocol improves sensitivity of détection of Mycobacterium tuberculosis in clinical samples by amplification of DNA. J. Clin. Microbiol. 29:712-717.
Saboor, S.A., N.M. Johnson, and J. McFadden. 1992. Detection of mycobacteria DNA in sarcoidis and tuberculosis with PCR. Lancet 339: 1012-1015.
Saiki, R.K., D.H. Gelfand, S. stoffel, S.J. Scarf, R. Higuchu, G.T. Horn, K.B. Mullis, and H.A. Erlich. 1988. Primer-directed enzymatic amplification of DNA with a thermostable DNA polymerase. Mol. Cell. Probes 5: 515-219.
Sambrook, J., E. P. Fritsch, and T. Maniatis. 1989. Molecular cloning: a laboratory manual, Cold Spring Harbor, NY.
Sauton, B. 1912. Sur la nutrition minérale du bacille tuberculeux. C.R. Acad. Sci Paris. 155:860.
Shankhar, S., N. Manjunath, K.K. Mohan, K. Praasad, M. Behari, G.K. Shriniwas, and K. Ahuja. 1991. Rapid diagnosis of tuberculosis meningitis by PCR. Lancet: 5-7.
Sj^bring, U., M. Mecklenburg, A.B. Andersen, and H. Mij^rner, 1990 PCR for detection of Mycobacterium tuberculosis. J. Bacteriol. 169:1080-1088.
Sudre, P., G. en Dam, and A. Kochi. 1992. Tuberculosis: a global overview of the situation today. Bull. W. H. O. 70: 149-159.
Telenti, A., F. Marchesi, M. Balz, F. Bally, E.C. Bottger, and T. Bodmer. 1993. Rapid identification of mycobacteria to the species level by polymerase chain reaction and restriction enzyme analysis. J. Clin. Microbiol. 31: 175-178.
van Soolingen, D., P.E.W. de Haas, P.W.M. Hermans, P.M.A. Groenen, and J.D.A. van Embden. 1993. Comparison of various repetitive DNA elements as genetic markers for strain differentiation and epidemiology of Mycobacterium tuberculosis. J. Clin. Microbiol. 31:1987-1995.
van Soolingen, D., P.E.W. de Haas, J. Haagsma, T. Eger, P.W.M. Hermans, V. Ritacco, A. Alito, and J.D.A. van Embden, 1994. Use of various genetic markers in differentiation of Mycobacterium bovis from animals and humans and for studying epidemiology of bovine tuberculosis. J. Clin. Microbiol. 32: 2425-2433.
Wren, B.W., Colby, S.M., Cubberley, R.R. and Pallen, M.J. 1992 Degenerate PCR primers for the amplification of fragments from genes encoding response regulators from a range of pathogenic bacteria FEMS Lett. 99, 287-292

## Revendications

1. Acide nucléique spécifique de mycobactéries appartenant au complexe *M. tuberculosis,* ayant une séquence nucléotidique choisie parmi la séquence SEQ ID N° 1, la séquence SEQ ID N° 2, et leurs séquences complémentaires.

2. Vecteur de clonage et/ou d'expression contenant une séquence d'acide nucléique selon la revendication 1.

3. Vecteur selon la revendication 2, **caractérisé en ce qu'**il s'agit du plasmide pRegX3Bcl ou PRegX3Mt1, respectivement déposés à la CNCM sous les numéros I-1765 et I-1766.

4. Sonde nucléotidique, de 24 à 218 nucléotides capable de s'hybrider spécifiquement à l'une des séquences selon la revendication 1 dans des conditions de forte stringence.

5. Sonde nucléotidique selon la revendication 4, **caractérisée en ce qu'**elle comprend la séquence SEQ ID N° 1 ou son brin complémentaire.

6. Sonde nucléotidique selon la revendication 5, **caractérisée en ce qu'**elle comprend deux séquences SEQ ID N° 1 successives, suivies d'une séquence SEQ ID N° 2.

7. Sonde nucléotidique de 24 à 218 nucléotides pour la détection de séquences d'acides nucléiques spécifiques de membres du complexe *M. tuberculosis* différents du BCG, **caractérisée en ce qu'**il s'agit d'une séquence correspondant à la région de la séquence SEQ ID N° 2 encadrant le codon GAG aux positions 40 à 42 ou de son brin complémentaire.

8. Sonde nucléotidique selon la evendication 7, **caractérisée en ce qu'**il s'agit d'une séquence comportant 9 paires de bases en amont et 9 paires de bases en aval du codon GAG aux positions 40 à 42 spécifique de la séquence SEQ ID N° 2.

9. Sonde nucléotidique selon la revendication 8, **caractérisée en ce qu'**il s'agit de la séquence SEQ ID N° 2 ou de son brin complémentaire.

10. Sonde nucléotidique selon la revendication 4, **caractérisée en ce qu'**elle est marquée par la digoxigénine.

11. Amorce nucléotidique d'au moins 19 nucléotides capable de s'hybrider spécifiquement à l'une des séquences selon la revendication 1 dans des conditions de forte stringence.

12. Paire d'amorces nucléotidiques d'au moins 19 nucléotides pour l'amplification d'une séquence nucléotidique spécifique de mycobactéries appartenant au complexe *M. tuberculosis,* comprenant :
a) une séquence nucléotidique correspondant à la séquence adjacente à la région intergénique senX3-regX3, dans la région 3' de senX3 et comprenant la séquence 5'GCGCGAGAGCCCGAACTGC3' ;
b) une séquence nucléotidique correspondant à la séquence adjacente à la région intergénique senX3-regX3, dans la région 5' de regX3 et comprenant la séquence 5'GCGCAGCAGAAACGTCAGC3'.

13. Paire d'amorces selon la revendication 12, **caractérisée en ce qu'**il s'agit de la paire d'amorces 5'GCGCGAGAGCCCGAACTGC3' et 5'GCGCAGCAGAAACGTCAGC3'.

14. Utilisation d'une séquence selon la revendication 1, pour la réalisation de sondes nucléotidiques de diagnostic ou d'amorces nucléotidiques utilisables dans une méthode d'amplification enzymatique.

15. Utilisation d'une sonde selon l'une quelconque des revendications 4 à 10 comme outil de détection ou de diagnostic *in vitro* de souches de mycobactéries appartenant au complexe *M. tuberculosis.*

16. Méthode de détection de souches de mycobactéries appartenant au complexe *M. tuberculosis* dans un échantillon biologique, comprenant les étapes suivantes :
(i) mise en contact de l'échantillon biologique avec une paire d'amorces selon la revendication 12 ou 13, dans des conditions permettant une hybridation desdites amorces aux acides nucléiques spécifiques de souches de mycobactéries appartenant au complexe *M. tuberculosis* ;
(ii) amplification desdits acides nucléiques;
(iii) mise en contact d'une sonde nucléotidique selon l'une quelconque des revendications 4 à 10 avec ledit échantillon biologique dans des conditions permettant la formation de complexes d'hybridation entre ladite sonde et les séquences d'acides nucléiques amplifiés ;
(iv) détection des complexes d'hybridation formés.

17. Méthode selon la revendication 16, **caractérisée en ce que** l'étape (iii) est réalisée avec une sonde nucléotidique selon la revendication 5.

18. Méthode de détection de la présence de membres du complexe *M. tuberculosis* autres que le BCG dans un échantillon biologique selon la revendication 16, **caractérisée en ce que** l'étape (iii) est réalisée avec une sonde nucléotidique selon la revendication 7.

19. Méthode de détection et de diagnostic différentiel du BCG et des autres membres du complexe *M. tuberculosis* dans un échantillon biologique, **caractérisée en ce qu'**on réalise une méthode de détection selon la revendication 17 et **en ce que** l'on recherche parmi les acides nucléiques amplifiés capables de former des complexes d'hybridation, ceux qui sont également capables de former des complexes d'hybridation avec une sonde nucléotidique selon la revendication 7.

20. Méthode selon la revendication 18 ou la revendication 19, pour différencier une infection par le BCG d'une infection par une mycobactérie virulente du complexe *M. tuberculosis* chez un sujet immunodéprimé.

21. Méthode selon la revendication 20, **caractérisée en ce que** le sujet immunodéprimé est un sujet infecté par le VIH.

22. Méthode pour l'identification de groupes de mycobactéries appartenant au complexe *M. tuberculosis,* **caractérisée en ce que** :
- on met en contact l'ADN desdites souches préalablement extrait avec une paire d'amorces selon la revendication 12 ou 13, dans des conditions de forte stringence permettant une hybridation spécifique des amorces à l'une des séquences selon la revendication 1 et l'obtention de produits d'amplification, et
- on mesure la longueur des produits d'amplification obtenus.

23. Méthode selon la revendication 22, **caractérisée en ce que** l'on utilise la paire d'amorces selon la revendication 13.

24. Trousse pour l'identification in vitro de souches de mycobactéries appartenant au complexe *M. tuberculosis* dans un prélèvement biologique comprenant:
- une paire d'amorces selon la revendication 12 ou 13 ;
- les réactifs nécessaires pour permettre l'amplification des séquences d'acides nucléiques spécifiques de souches appartenant au complexe *M. tuberculosis à* l'aide desdites amorces,
- éventuellement des moyens pour révéler les fragments amplifiés.

## Patentansprüche

1. Nucleinsäure, welche für Mykobakterien, die zum *M.-tuberculosis*-Komplex gehören, spezifisch ist und eine Nucleotidsequenz besitzt, die aus der Sequenz SEQ ID Nr. 1, der Sequenz SEQ ID Nr. 2 und deren komplementären Sequenzen ausgewählt ist.

2. Klonierungs- und/oder Expressionsvektor, der eine Nucleinsäuresequenz nach Anspruch 1 enthält.

3. Vektor nach Anspruch 2, **dadurch gekennzeichnet, dass** es sich um das Plasmid pRegX3Bcl bzw. PRegX3Mt1 handelt, die beim CNCM unter der Nummer I-1765 bzw. I-1766 hinterlegt sind.

4. Nucleotidsonde mit 24 bis 218 Nucleotiden, die in der Lage ist, mit einer der Sequenzen nach Anspruch 1 unter hoch stringenten Bedingungen spezifisch zu hybridisieren.

5. Nucleotidsonde nach Anspruch 4, **dadurch gekennzeichnet, dass** sie die Sequenz SEQ ID Nr. 1 oder deren komplementären Strang umfasst.

6. Nucleotidsonde nach Anspruch 5, **dadurch gekennzeichnet, dass** sie zwei aufeinander folgende Sequenzen SEQ ID Nr. 1, auf welche eine Sequenz SEQ ID Nr. 2 folgt, umfasst.

7. Nucleotidsonde mit 24 bis 218 Nucleotiden für die Detektion von Nucleinsäuresequenzen, die für Mitglieder des *M*.*-tuberculosis*-Komplexes, die kein BCG sind, spezifisch sind, **dadurch gekennzeichnet, dass** es sich um eine Sequenz, die der Region der Sequenz SEQ ID Nr. 2 entspricht, die das Codon GAG auf den Loci 40 bis 42 einrahmt, oder um ihren komplementären Strang handelt.

8. Nucleotidsonde nach Anspruch 7, **dadurch gekennzeichnet, dass** es sich um eine Sequenz handelt, die stromaufwärts und stromabwärts vom Codon GAG auf den Loci 40 bis 42, das für die Sequenz SEQ ID Nr. 2 spezifisch ist, jeweils 9 Basenpaare enthält.

9. Nucleotidsonde nach Anspruch 8, **dadurch gekennzeichnet, dass** es sich um die Sequenz SEQ ID Nr. 2 oder deren komplementären Strang handelt.

10. Nucleotidsonde nach Anspruch 4, **dadurch gekennzeichnet, dass** sie mit Digoxigenin markiert ist.

11. Nucleotidprimer mit mindestens 19 Nucleotiden, der in der Lage ist, mit einer der Sequenzen nach Anspruch 1 unter hoch stringenten Bedingungen spezifisch zu hybridisieren,

12. Nucleotidprimerpaar mit mindestens 19 Nucleotiden zur Amplifizierung einer Nucleotidsequenz, die für Mykobakterien, die zum *M.*-*tuberculosis*-Komplex gehören, spezifisch ist, das
a) eine Nucleotidsequenz, die der Sequenz entspricht, die an den intergenischen Bereich senX3-regX3 in der Region 3' von senX3 angrenzt und die Sequenz 5'GCGCGAGAGCCCGAACTGC3' umfasst, und
b) eine Nucleotidsequenz, die der Sequenz entspricht, die an den intergenischen Bereich senX3-regX3 in der Region 5' von regX3 angrenzt und die Sequenz 5'GCGCAGCAGAAACGTCAGC3' umfasst,
umfasst.

13. Primerpaar nach Anspruch 12, **dadurch gekennzeichnet, dass** es sich um das Primerpaar 5'GCGCGAGAGCCCGAACTGC3' und 5'GCGCAGCAGAAACGTCAGC3' handelt.

14. Verwendung einer Sequenz nach Anspruch 1 zur Herstellung von Diagnosenucleotidsonden oder Nucleotidprimern, die in einem enzymatischen Amplifizierungsverfahren verwendbar sind.

15. Verwendung einer Sonde nach einem der Ansprüche 4 bis 10 als Werkzeug zur *in-vitro*-Detektion oder -Diagnose von Mykobakterienstämmen, die zum *M*.-*tuberculosis*-Komplex gehören.

16. Verfahren zur Detektion von zum *M*.-*tuberculosis*-Komplex gehörenden Mykobakterienstämmen in einer biologischen Probe, das die Stufen:
I) In-Berührung-Bringen der biologischen Probe mit einem Primerpaar nach Anspruch 12 oder 13 unter Bedingungen, die eine Hybridisierung dieser Primer mit Nucleinsäuren erlauben, die für zum M.-tuberculosis-Komplex gehörende Mykobakterienstämme spezifisch sind,
II) Amplifizierung dieser Nucleinsäuren,
III) In-Berührung-Bringen einer Nucleotidsonde nach einem der Ansprüche 4 bis 10 mit der biologischen Probe unter Bedingungen, welche die Bildung von Hybridisierungskomplexen dieser Sonde mit den amplifizierten Nucleinsäuresequenzen erlauben, und
IV) Detektion der gebildeten Hybridisierungskomplexe
umfasst.

17. Verfahren nach Anspruch 16, **dadurch gekennzeichnet, dass** die Stufe III) mit einer Nucleotidsonde nach Anspruch 5 durchgeführt wird.

18. Verfahren zur Detektion des Vorhandenseins von Mitgliedern des *M.-tuberculosis*-Komplexes, die kein BCG sind, in einer biologischen Probe nach Anspruch 16, **dadurch gekennzeichnet, dass** die Stufe III) mit einer Nucleotidsonde nach Anspruch 7 durchgeführt wird.

19. Verfahren zur Detektion und Differentialdiagnose von BCG und den anderen Mitgliedern des *M*.-*tuberculosis*-Komplexes in einer biologischen Probe, **dadurch gekennzeichnet, dass** ein Detektionsverfahren nach Anspruch 17 durchgeführt wird, **und dass** von den amplifizierten Nucleinsäuren, die in der Lage sind, Hybridisierungskomplexe zu bilden, diejenigen herausgesucht werden, die auch in der Lage sind, Hybridisierungskomplexe mit einer Nucleotidsonde nach Anspruch 7 zu bilden,

20. Verfahren nach Anspruch 18 oder 19, um bei einem Lebewesen mit geschwächter Immunabwehr eine Infektion durch BCG von einer Infektion durch ein virulentes Mycobacterium des *M.-tuberculosis*-Komplexes zu unterscheiden.

21. Verfahren nach Anspruch 20, **dadurch gekennzeichnet, dass** das Lebewesen mit geschwächter Immunabwehr mit HIV infiziert ist.

22. Verfahren zur Identifizierung von Gruppen von Mykobakterien, die zum *M.-tuberculosis*-Komplex gehören, **dadurch gekennzeichnet, dass**
- die DNA dieser Stämme, die zuvor extrahiert worden ist, mit einem Primerpaar nach Anspruch 12 oder 13 unter hoch stringenten Bedingungen, die eine spezifische Hybridisierung der Primer mit einer der Sequenzen nach Anspruch 1 und das Erhalten von Amplifikationsprodukten erlauben, in Berührung gebracht und
- die Länge der erhaltenen Amplifikationsprodukte gemessen wird.

23. Verfahren nach Anspruch 22, **dadurch gekennzeichnet, dass** das Primerpaar nach Anspruch 13 verwendet wird.

24. Kit zur *in*-*vitro*-Identifizierung von zum *M.-tuberculosis*-Komplex gehörenden Mykobakterienstämmen in einer biologischen Probe, welcher
- ein Primerpaar nach Anspruch 12 oder 13,
- die erforderlichen Reagenzien, um die Amplifizierung der Nucleinsäuresequenzen, die für Stämme spezifisch sind, die zum *M.*-*tuberculosis*-Komplex gehören, durch diese Primer zu ermöglichen, und
- gegebenenfalls Mittel zum Nachweis der amplifizierten Fragmente
umfasst.

## Claims

1. Nucleic acid specific to mycobacteria belonging to the *M. tuberculosis* complex, having a nucleotide sequence selected from the sequence SEQ ID No. 1, the sequence SEQ ID No. 2, and their complementary sequences.

2. Cloning and/or expression vector containing a sequence of nucleic acids according to Claim 1.

3. Vector according to Claim 2, **characterized in that** it is the plasmid pRegX3Bc1 or PRegX3Mt1 respectively deposited at the CNCM under the numbers I-1765 and I-1766.

4. Nucleotide probe having from 24 to 218 nucleotides and capable of hybridizing specifically with one of the sequences according to Claim 1 under conditions of great stringency.

5. Nucleotide probe according to Claim 4, **characterized in that** it comprises the sequence SEQ ID No. 1 or its complementary strand.

6. Nucleotide probe according to Claim 5, **characterized in that** it comprises two successive sequences SEQ ID No. 1, followed by a sequence SEQ ID No. 2.

7. Nucleotide probe having from 24 to 218 nucleotides, for the detection of specific sequences of nucleic acids of members of the *M. tuberculosis* complex which are different from BCG, **characterized in that** it is a sequence corresponding to the region of the sequence SEQ ID No. 2 surrounding the GAG codon in the positions 40 to 42 or of its complementary strand.

8. Nucleotide probe according to Claim 7, **characterized in that** it is a sequence comprising 9 base pairs upstream and 9 base pairs downstream of the GAG codon in the specific positions 40 to 42 of the sequence SEQ ID No. 2.

9. Nucleotide probe according to Claim 8, **characterized in that** it is the sequence SEQ ID No. 2 or its complementary strand.

10. Nucleotide probe according to Claim 4, **characterized in that** it is labelled by digoxygenin.

11. Nucleotide primer having at least 19 nucleotides and capable of hybridizing specifically with one of the sequences according to Claim 1 under conditions of great stringency.

12. Pair of nucleotide primers having at least 19 nucleotides, for the amplification of a nucleotide sequence specific to mycobacteria belonging to the *M. tuberculosis* complex, comprising:
a) a nucleotide sequence corresponding to the sequence adjacent to the intergenic region senX3-regX3, in the region 3' of senX3 and comprising the sequence 5'GCGCGAGAGCCCGAACTGC3';
b) a nucleotide sequence corresponding to the sequence adjacent to the intergenic region senX3-regX3, in the region 5' of regX3 and comprising the sequence 5'GCGCAGCAGAAACGTCAGC3'.

13. Pair of primers according to Claim 12, **characterized in that** it is the pair of primers 5'GCGCGAGAGCCCGAACTGC3' and 5'GCGCAGCAGAAACGTCAGC3'.

14. Use of a sequence according to Claim 1, for the production of diagnostic nucleotide probes or of nucleotide primers which can be used in an enzymatic amplification method.

15. Use of a probe according to any one of Claims 4 to 10 as an *in vitro* tool for detection or for diagnosis of strains of mycobacteria belonging to the *M. tuberculosis* complex.

16. Method of detection of strains of mycobacteria belonging to the *M. tuberculosis* complex in a biological sample, comprising the following steps:
(i) contacting the biological sample with a pair of primers according to Claim 12 or 13 under conditions allowing hybridization of the said primers to the specific nucleic acids of strains of mycobacteria belonging to the *M. tuberculosis* complex;
(ii) amplification of the said nucleic acids;
(iii) contacting a nucleotide probe according to any one of Claims 4 to 10 with the said biological sample under conditions allowing the formation of hybridization complexes between the said probe and the amplified sequences of nucleic acids;
(iv) detection of the hybridization complexes formed.

17. Method according to Claim 16, **characterized in that** step (iii) is carried out with a nucleotide probe according to Claim 5.

18. Method of detection of the presence of members of the *M. tuberculosis* complex other than BCG in a biological sample according to Claim 16, **characterized in that** step (iii) is carried out with a nucleotide probe according to Claim 7.

19. Method of detection and of differential diagnosis of BCG and of other members of the *M. tuberculosis* complex in a biological sample, **characterized in that** a detection method according to Claim 17 is carried out and **in that** a search is made among the amplified nucleic acids capable of forming hybridization complexes for those which are likewise capable of forming hybridization complexes with a nucleotide probe according to Claim 7.

20. Method according to Claim 18 or Claim 19 for differentiating an infection by BCG from an infection by a virulent mycobacterium of the *M. tuberculosis* complex in an immunodeficient subject.

21. Method according to Claim 20, **characterized in that** the immunodeficient subject is a subject infected with HIV.

22. Method for the identification of groups of mycobacteria belonging to the *M. tuberculosis* complex, **characterized in that**:
- the DNA of the said strains previously extracted with a pair of primers according to Claim 12 or 13 is contacted under conditions of great stringency allowing a specific hybridization of the primers with one of the sequences according to Claim 1 and the obtainment of amplification products, and
- the length of the amplification products obtained is measured.

23. Method according to Claim 22, **characterized in that** the pair of primers according to Claim 13 is used.

24. Kit for the in vitro identification of strains of mycobacteria belonging to the *M. tuberculosis* complex in a biological sample comprising:
- a pair of primers according to Claim 12 or 13;
- the reagents necessary to allow the amplification of the sequences of nucleic acids specific to strains belonging to the *M. tuberculosis* complex using the said primers,
- optionally, means for revealing the amplified fragments.


### LISTE DE SEQUENCES

(1) INFORMATION GENERALE:
   (i) DEPOSANT:
      (A) NOM: Institut Pasteur de Lille
      (B) RUE: 1 rue du Professeur Calmette
      (C) VILLE: Lille
      (E) PAYS: France
      (F) CODE POSTAL: 59019
      (G) TELEPHONE: 0320877800
      (H) TELECOPIE: 0320877906

      (A) NOM: Institut National de la Santé et de la recherche médicale
      (B) RUE: 101 rue de Tolbiac
      (C) VILLE: Paris
      (E) PAYS: France
      (F) CODE POSTAL: 75013
      (G) TELEPHONE: 0144236039
      (H) TELECOPIE: 0145850766
   (ii) TITRE DE L' INVENTION: fragments d'acides nucléiques spécifiques de M. tuberculosis
   (iii) NOMBRE DE SEQUENCES: 2
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 77 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Mycobacterium tuberculosis
   (xi) DESCRIPTION DE LA SEQUENCE: SEQ ID NO: 1:
   ATGACCTGCG CCGACGACGA TGCAGAGCGT AGCGATGAGG TGGGGGCACC ACCCGCTTGC    60
   GGGGGAGAGT GGCGCTG                                                   77
(2) INFORMATION POUR LA SEQ ID NO: 2:
   (i) CARACTERISTIQUES DE LA SEQUENCE:
      (A) LONGUEUR: 53 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Mycobacterium tuberculosis
   (xi) DESCRIPTION DE LA SEQUENCE: SEQ ID NO: 2:
   ATGACCTGCG CCGACGACGA TGCAGAGCGT AGCGATGAGG AGGAGTGGCG CTG          53